# EUROPEAN PATENT APPLICATION

(11) **EP 2 510 896 A1**
(43) Date of publication of application: **17.10.2012**
(21) Application number: 11162738.6
(22) Date of filing: 15.04.2011
(51) Int. Cl.: A61B 19/00

(54) **Container for an organ**

(71) Applicant: Universität Bern, 3012 Bern (CH)
(72) Inventor: The designation of the inventor has not yet been filed
(74) Representative: Schmauder & Partner AG Patent- & Markenanwälte VSP

(57) **Abstract**

The invention relates to a method and a system for manufacturing a container (1) for an organ. An image data set of the organ is collected using a medical image acquisition apparatus, in particular a CT apparatus (CT: computer tomography), a MRI apparatus (MRI: magnetic resonance imaging), and/or an ultrasonography apparatus. A surface shape of the organ or parts thereof is determined by analyzing the collected image data set. The container (1) is manufactured in accordance to the determined surface shape of the organ, such that the organ as a whole or only partially may be stabilized and shaped within the container (1) during a medical intervention.

## Description

### Technical Field

The invention relates to a method and a system for manufacturing a container for an organ.

### Background Art

For the treatment of diseases, efficient medical interventions like the surgery of organs are of highest importance. In such surgical procedures, the body of a patient may be opened in order to access the organ to be treated. Organs to be treated may include the lung, the heart, the liver, the pancreas, the kidney, the intestine or any other organ of a body. For example, in case of a cancer in an organ, respective tissue has to be removed from the organ, for example by cutting away parts of the organ or by ablation procedures. On the one hand, time spent during surgical procedures on an organ has to be as short as possible, such that a body of a person is exposed to the surgery for as little time as possible. On the other hand, medical interventions like surgical procedures have to be as precise as possible, such that as much as healthy tissue as possible is preserved, for example in case of a cancer to be removed from an organ.

Prior to surgical procedures, the patient is given a medical examination involving pre-operative tests and assessments. Such medical examinations may include gathering X-ray images of parts of the body. For example, images of the lung may be collected in order to locate a lung cancer. Thereafter, during the surgery, the body of the patient is opened and the respective organ is accessed, for example the lung in order to remove parts of the lung containing the cancer as determined by the X-ray images collected during the medical examination. In order to remove all of the cancer, a safety margine is maintained. The safety margin may be minimized by collecting further images of the organ during the surgery, for example. Hence, in order to determine the location of the cancer more precisely and therefore to minimize the safety clearance, an ultrasound device or any other medical imaging device may be used during the medical intervention.

Open heart surgery is difficult because of the inaccessibility of the field of operation on the heart and the difficulty of maintaining the heart in place in a steady position. US 4,637,377 (Loop) relates to a surgical pillow for supporting the heart or another organ during surgical operations. The pillow is deformable by external forces applied from the organ. The surface of the pillow is of a character preventing slippage of the organ without causing irritation or contraction of the organ. The inner body member of the pillow may comprise an aggregate of synthetic plastic foam material, such as micro-balloons.

WO 01/43654 (LB Medical) relates to organ navigation by referencing between a data set describing the spatial model of a body and the real physical environment in which the body is located. A position referencing body is placed in the real body. The position referencing body includes one or more elementary bodies (markers) that can be spatially and sensorially detected. Thus, a rigid organ or part thereof can be localized in space by means of a surface scanning device that is able to detect said referencing bodies during the scan and utilize its positions during the referencing/registration process.

In currently conducted surgical procedures, precision may be improved to some extent through image guidance technology. However, deploying such technology is complicated and resource consuming. Typically, more time is needed to perform an image-guided surgical procedure, leading to additional burden for the patient.

### Summary of the invention

It is the object of the invention to create a method and a system for improving the efficiency of a medical intervention in or on an organ pertaining to the technical field initially mentioned, wherein a container for an organ is manufactured to achieve the improved efficiency during the medical intervention, in particular an improved precision during a surgical procedure or intervention at the organ and a minimized time necessary to perform the surgery.

The solution of the invention is specified by the features of claim 1. According to the invention a method for manufacturing a container for an organ comprises:
a) collecting an image data set of the organ using a medical image acquisition apparatus, in particular a CT apparatus (CT: computer tomography), a MRI apparatus (MRI: magnetic resonance imaging), and/or an ultrasonography apparatus;
b) determining a surface shape of the organ or parts thereof by analyzing the collected image data set; and
c) manufacturing the container in accordance to the determined surface shape of the organ, such that the organ as a whole or only partially may be stabilized and shaped within the container during a medical intervention.

During the surgery, the organ is placed inside the container. As the container is manufactured in accordance to the surface shape of the organ, the container forms a wrapping to the organ which is in correspondence to the physical dimensions of the organ, in particular exactly in the known shape during medical examination of the patient and according to the collected image data before opening the body and accessing the organ, which typically leads to a deformation of the organ and a re-orientation of structures included in the organ. The container may be manufactured such that only a small gap or no gap at all is between the container and the organ. Moreover, the container may be manufactured such that it has a required stiffness, in order to sufficiently stabilize the organ during the medical intervention or the surgery. Based on the determined surface shape, a three-dimensional virtual container may be calculated. Spatial, morphological, or functional properties of the virtual model may be intentionally added, removed, or enhanced. The container may be manufactured in accordance to the calculated virtual model.

The container may include openings, such that required surgical instruments may be inserted through the openings in order to perform a desired medical intervention on the organ. The container may also serve to provide reference points, such that particular locations of the organ, for example parts that have to be removed, may be easily referenced to. For example, referencing of anatomical structures on or within the organ arranged in the container may be performed by triangulation from points on the surface of the container and therefore a reference between the organ and the medical image may be provided.

Any kind of medical imaging apparatus may be used in order to collect image data of the organ. In particular, a CT apparatus, a MRI apparatus, possibly connected to a PET or SPECT scanning device, and/or an ultrasonography apparatus may be used. It is further possible to utilize surface scanners based on laser or structured light or similar means. The medical imaging apparatus may be directed to the collection of multidimensional data, in particular to the collection of 2D/3D/4D image data of the organ. For example, a 3D image data set may include the spatial distribution of a density of the organ, whereas a 4D image data set may include the spatial distribution of the density of the organ with respect to time, which forms a space-time geometry. A 4D image data set may be in particular advantageous for organs which change the geometry of their surface with time largely, like for example the heart. But also for organs with small variations of their geometry, a 4D image set may provide a more accurate basis for manufacturing the container for the organ, because the dimensions of the container are manufactured according to a minimal or maximal geometry of the organ, which enables a more accurate stabilization of the organ during a medical intervention.

Preferably, several image data sets of organs of several persons are collected, wherein standardized surface shapes of organs are determined and containers of standardized and predefined sizes and/or shapes are manufactured accordingly, such that a container of a certain size and/or shape may be selected in order to stabilize the organ of a certain person during a surgery. For different patient cohorts or populations of humans the size and/or shape of a certain organ, for example the liver, typically varies within certain ranges. Hence, data sets of a representative human subpopulation may be acquired and descriptive statistical parameters such as mean, min, max, standard deviations, Eigen modes or similar parameters may be derived with respect to the shape, size, or other distributions of the organ or substructures contained in the organ. Statistical surface shapes of organs are determined (e.g. a mean shape) and containers of standardized shapes are manufactured, such that standardized containers with shapes derived from a population of humans are provided. For example, if the variations of the statistical parameters are large, a larger number of standardized containers may be manufactured, whereas in the case when the variations are small, a few standardized containers may be sufficient to cover all possible sizes of the organ. In order to select a container for an organ of a certain patient, image data of the organ of the patient may additionally be collected before the medical intervention, such that a container with a known and predetermined accuracy is available during the medical intervention. Standardized containers may provide a cost effective solution to improve the efficiency of a medical intervention, when compared to individually manufactured containers.

Alternatively, the container for an organ is custom-made and is manufactured in an individual shape and size. This is especially required for an organ that does not have typical dimensions and varies largely within the human population. In case of a custom-made container, additional mechanical structures that allow for the guiding of surgical and interventional instruments in predefined degrees of freedom may be added and enable surgery according to a predefined plan, which further enhances the efficiency, in particular with respect to precision and time.

In a preferred embodiment, manufacturing the container includes a conventional or rapid prototyping process, including but not limited to laser sintering, vacuum casting, stereolithography, 3D printing and/or fused deposition modelling. Laser sintering is an additive manufacturing technique that uses a high power laser (e.g. a carbon dioxide laser) to fuse small particles of plastic, metal, ceramic, or glass powders into a mass that has a desired 3-dimensional shape. In vacuum casting, for example a silicone cast is produced based on a master positive model, which has been built with one of the new generative technologies or in a conventional way. The cast is then filled with a two-part polyurethane resin under vacuum. Stereolithography is an additive manufacturing process using a vat of liquid energy (i.e. UV light-) curable photopolymer "resin" and an energy source that delivers defined amounts of energy to predefined positions to create physical parts layer by layer at a time. 3D printing is an additive manufacturing technology where a three dimensional object is created by laying down and connecting (i.e. gluing) successive layers of material. Fused deposition modelling is an additive manufacturing technology commonly used for modelling, prototyping, and production applications.

Any technology may be used in order to manufacture the container, either a container according to a standardized size or a container according to a custom-made size.

Preferably, the container is manufactured in a biocompatible material, in particular a metallic material or a polymer. The material allows for the application on or within the certain parts of the body. Moreover, the material has to maintain a mechanical stability suitable to the order magnitude required in accuracy during the actual medical intervention. Metallic materials include but are not limited to titanium, stainless steel or alloys thereof. Polymers may include polyether ether ketone (PEEK) material. Any combination of these materials and/or any other material suitable for medical interventions or surgical applications may be used.

In a preferred embodiment, the surface shape of the organ is reduced to a mesh, wherein a corresponding container is manufactured in a mesh structure. The reduction to a mesh may relate to a virtual mesh calculated using a computer software. The mesh may be selected according to a mesh structure defined by the shape, size, isotropy, or orientation. The mesh may include thin, for example 1-2mm, structural elements that are connected to each other. Any other parameter may be used to define the mesh structure. The mesh structure has the advantage that a sufficient stability of the container may be provided at the same time as openings in the container in order to perform a medical intervention on an organ contained in the container. Moreover, various parts of the mesh structure, for example an edge or a crossing, may serve as reference points with respect to the organ or structures contained inside the organ. Such reference points may improve precision of a medical intervention.

Preferably, the container is manufactured such that only parts of the organ are covered and/or that the container is manufactured such that extra openings for accessing the organ during a medical intervention are provided. For example only the left or the right lobe may be covered by the container. This may be useful in case blood vessels or other tissue attached to the organ have to remain uncovered such that the vessels or other tissue are not damaged when the organ is placed in the container. The extra openings may serve as an access to the organ, for example for ultrasound scanning or instrument manipulation.

In a preferred embodiment, the container is manufactured such that it includes several parts, which are connectable with one or more adapters or fittings. Hence, the container can be assembled from two or more individual parts, which are inter-connectable in a reproducible and defined manner. Accordingly, during a medical intervention, the container is assembled together at the location of the organ, in order to place and stabilize the organ step by step in the container.

Preferably, the container is manufactured such that it includes reference elements which are detectable with a surgical instrument guidance system, in particular in order to monitor the position and/or the orientation of a surgical instrument relative to the container and therefore relative to the organ arranged in the container during a medical intervention. The reference elements may be designed to be detectable by an optical and/or electromagnetic tracking system. With the aid of surgical instrument guidance systems efficiency can be further improved, in particular with respect to precision and/or time needed to perform the medical intervention.

The container may be manufactured such that it contains elements that allow for simple identification of said elements in a medical image data set. Thus, when the container is in place at the organ and an additional (i.e. an intraoperative) medical image recording is conducted, the referential elements can be used to automatically calculate the spatial transformation between the coordinate system of the patient and that of the medical image data set (a.k.a. automatic registration).

In a preferred embodiment, the container is manufactured such that it includes one or more guiding mechanisms for surgical instruments and/or for diagnostic equipment. In a medical examination prior to a medical intervention, image data of the organ of a patient may be collected and analyzed, for example with respect to the exact location of structures like cancer lesions inside the organ. Prior to the medical intervention, the container is manufactured according to the collected image data, such that medical instruments like an ablation needle are guided by the guiding mechanism in a predefined position and orientation, for example to ablate a cancer inside the organ. The guiding mechanism may include guiding holes and/or longitudinal recesses for guiding saws, blades or the like. Moreover, the guiding mechanism may be arranged for the guiding, correlation and/or interaction with diagnostic and medical imaging equipment such as, for example, a sonographic probe of a sonography imaging equipment. In particular, the guiding mechanism may be arranged according to a shape of a transducer, such that efficiency in the handling of the transducer is improved. Hence, the guiding mechanism further improves the efficiency, in particular with respect to precision and time used for a medical intervention like a surgical procedure to remove cancer tissue from an organ.

A system for manufacturing a container for an organ comprises:
a) an image data acquisition apparatus designed to collect an image data set of the organ, in particular a CT apparatus (CT: computer tomography), a MRI apparatus (MRI: magnetic resonance imaging), and/or an ultrasonography apparatus;
b) a surface shape determination module for determining a surface shape of the organ or parts thereof by analyzing the collected image data set; and
c) a manufacturing apparatus for manufacturing the container in accordance to the determined surface shape of the organ, such that the organ as a whole or only partially may be stabilized and shaped within the container during a surgery.

Image data acquisition apparatuses are widely available for various medical indications and qualities on the market. The surface shape determination module may be implemented as a software module which may be executed on a microprocessor system. The software module may be programmed in any software language, like C or C++, and provide a software based manual, a semi-automatic or an automatic determination of the surface shape. The surface shape determination module may be designed to output a 3D data set describing the surface shape of the organ. This 3D data set may be transferred to the manufacturing apparatus for manufacturing the container, for example through a data interface like an Ethernet interface. In a manufacturing step, the container is manufactured and then used during a medical intervention in order to stabilize and shape an organ, such that the medical intervention can be performed with higher precision and within a shorter time as well.

In a preferred embodiment, the image data acquisition apparatus, the surface shape determination module and the manufacturing apparatus are designed such that several image data sets of organs of several persons are collected, wherein standardized surface shapes of organs are determined and containers of standardized and predefined sizes and/or shapes are manufactured accordingly, such that a container of a certain size and/or shape may be selected in order to stabilize the organ of a certain person during a surgery. Standardized containers may provide a cost effective solution to improve the efficiency of a medical intervention.

Alternatively, the system is arranged such that custom-made containers may be manufactured, which has the advantage that the containers are optimally adapted to the needs of a patient.

In a preferred embodiment, the manufacturing apparatus is designed to manufacture the container in a conventional or rapid prototyping process, including but not limited to laser sintering, vacuum casting, stereolithography, 3D printing and/or fused deposition modelling. This has the advantage that costs and time to manufacture the container may be limited to a desired level.

Preferably, the manufacturing apparatus is designed to manufacture the container such that it includes several parts, which are connectable with one or more adapters or fittings. This has the advantage that placement of the organ into the container is simplified.

In a preferred embodiment, the manufacturing apparatus is designed to manufacture the container such that it includes reference elements which are detectable with a surgical instrument guidance system, in particular in order to monitor the position and/or orientation of a surgical instrument relative to the container and therefore relative to the organ arranged in the container during a medical intervention. This has the advantage that with the aid of a surgical instrument guidance system the efficiency of the medical intervention is increased, in particular the precision is increased and the time needed to perform the medical intervention is reduced.

Preferably, the manufacturing apparatus is designed to manufacture the container such that it includes one or more guiding mechanisms for surgical instruments and/or for diagnostic equipment. This has the advantage that the efficiency of a medical intervention is increased, because the guiding mechanism enables for example to perform a surgery faster and with higher precision, as the surgical instruments and/or the diagnostic equipment are brought into an optimal position with respect to the organ.

Other advantageous embodiments and combinations of features come out from the detailed description below and the totality of the claims.

### Brief description of the drawings

The drawings used to explain the embodiments show:
- Fig. 1: a container 1 according to the invention;
- Fig. 2: a container 1 including reference elements 5;
- Fig. 3: a container 1 including a guiding mechanism 6;
- Fig. 4: a container 1 subdivided into 4 parts 1.1, 1.2, 1.3, 1.4;
- Fig. 5: a container 1 in a perspective view; and
- Fig. 6: a container 1 subdivided into 3 parts 1.1, 1.2, 1.3 in a perspective view.

In the figures, the same components are given the same reference symbols.

### Preferred embodiments

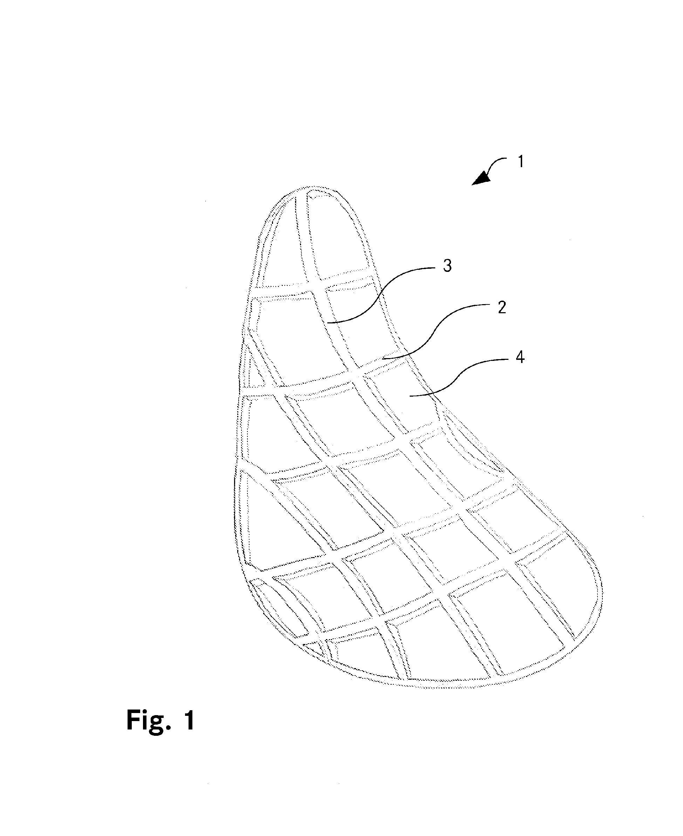

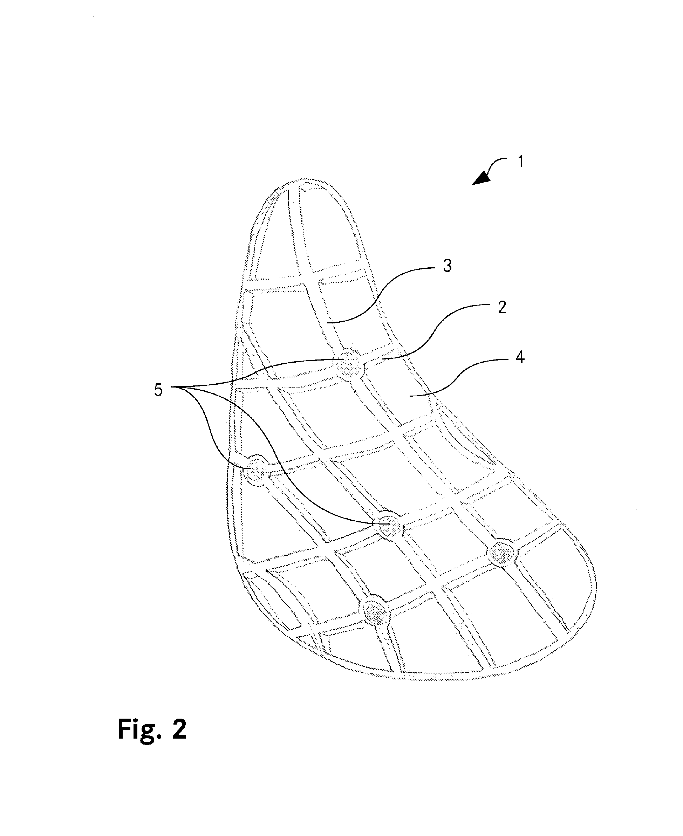

Fig. 1 shows schematically a container 1 for an organ. The container 1 has been manufactured by collecting image data of an organ of a patient, by determining the surface shape of the organ, for example the liver, and by manufacturing the container 1 according to the surface shape of the organ. For better understanding of the drawings, only a part of the container 1 is shown in Fig. 1 and in the Fig. 2-4 which are described below.

The container 1 has an elongate form extending in a longitudinal direction and in a transversal direction. Typically, the container 1 is longer in longitudinal direction than in transversal direction. Moreover, the container 1 has a mesh structure. In a longitudinal direction of the container 1, longitudinal mesh elements 3 extend from one end of the container 1 to the other. In a transversal direction, transversal mesh elements 2 extend transversally between the ends of the container 1. Between the longitudinal mesh elements 3 and the transversal mesh elements 2, the container 1 has apertures 4.

The thickness of the longitudinal mesh elements 3 and the transversal mesh elements 2 is chosen such that a required stiffness of the container 1 is achieved. For example, the longitudinal mesh elements 3 and the transversal mesh elements 2 have a thickness of about 1-2 mm. The distance between the longitudinal mesh elements 3 and the transversal mesh elements 2 is chosen such that accessibility of an organ arranged in the container 1 and stability of the container 1 is optimized.

The container 1 may be manufactured using any biocompatible material, for example a metallic material like titanium or stainless steel, a polymer like polyether ether ketone (PEEK), and/or any combination thereof. Manufacturing the container 1 may include any manufacturing process, for example a rapid prototyping process.

Fig. 2 shows schematically a container 1 according to the invention with longitudinal mesh elements 3 and transversal mesh elements 2, wherein apertures 4 are provided between the mesh elements. Additionally, reference elements 5 are arranged on the container 1. The reference elements 5 may be arranged at any desired location of the container 1, for example at the crossings of the longitudinal mesh elements 3 and the transversal mesh elements 2. The reference elements 5 may include optical and/or electromagnetic components, such that a surgical instrument guidance system may detect the exact location and orientation of the container 1. With the aid of the surgical instrument guidance system, medical instruments may be guided during a medical intervention according to a planned procedure. Guiding the medical instruments may be performed manually by a person and the surgical instrument guidance system may provide an appropriate acoustic and/or visual feedback to the person. Alternatively, the medical instruments may be guided fully automatically by a robot. Semi-automatic systems may include a robot which supports a human to guide a medical instrument.

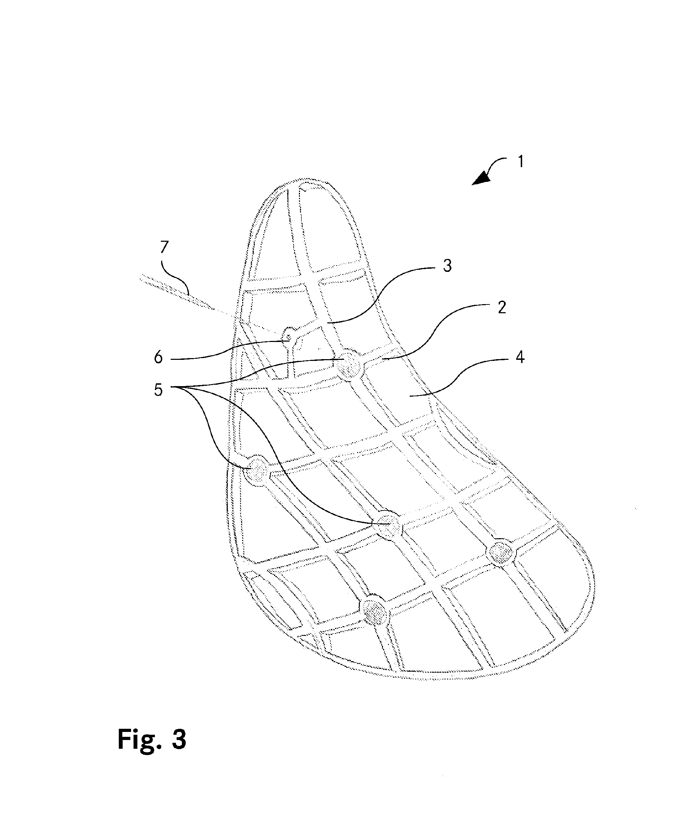

Fig. 3 shows schematically a container 1 according to Fig. 2. Additionally, a guiding mechanism 6 is provided, in order to precisely guide a medical instrument 7. The guiding mechanism is arranged at the mesh structure of the container 1 and may include a guiding hole, for example, designed to guide a medical instrument like a needle in an appropriate position and direction. The guiding hole may be sufficiently long, such that a needle inserted into the guiding hole is directed into a desired direction with little clearance. The guiding hole is designed according to collected image data of the organ, for example according to the location of a cancer in the organ. Hence, in a surgical procedure, the needle is inserted into the guiding hole and moved through the guiding hole according to a predefined length. For example, a coloured marking may be arranged on the needle, wherein the needle is moved forward through the guiding hole until the coloured marking flushes with the guiding hole. Accordingly, the tip of the needle is precisely at a predefined location inside the organ, namely the location where cancer tissue has to be removed. Removing the cancer tissue may be performed using an ablation process, for example.

As shown in Fig. 2, both a guiding mechanism 6 and reference elements 5 may be arranged on the container 1. However, depending on the medical intervention to be performed, only a guiding mechanism 6 may be arranged on the container 1, without the arrangement of reference elements 5.

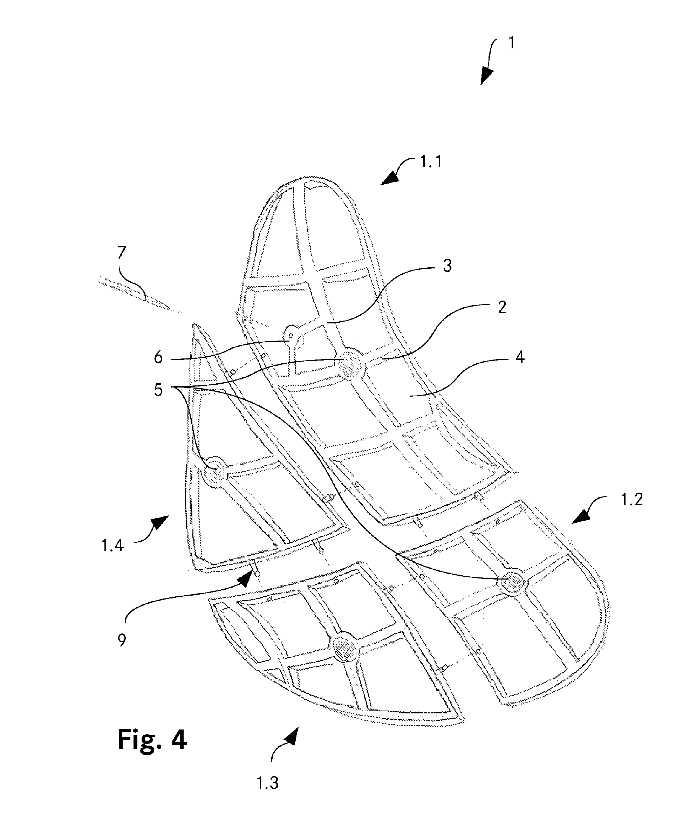

Fig. 4 shows schematically a container 1 which includes 4 container parts 1.1, 1.2, 1.3, 1.4. The container 1 may be subdivided in any number of container parts. The container parts 1.1, 1.2, 1.3, 1.4 are designed such that they can be assembled together with one or more adapters or fittings 9. Any kind of known adapters or fittings 9 may be used, for example bolts, rivets, clamps, clips, or any other kind of adapters or fittings. The size of the parts 1.1, 1.2, 1.3, 1.4 may be designed such that the container 1 can be easily assembled and the organ easily arranged in the container 1 during a medical intervention. Moreover, the parts 1.1, 1.2, 1.3, 1.4 may be designed such that the container 1 may be opened by pivoting or swinging away one of the parts in order to have a larger access area to the organ arranged in the container 1.

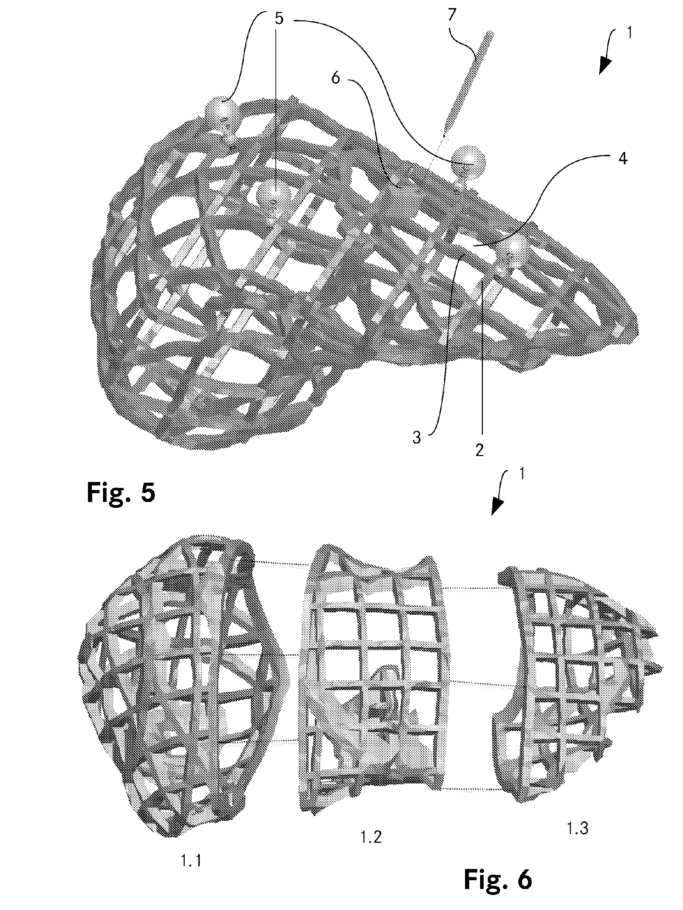

Fig. 5 shows schematically a container 1 in a perspective view. As in the previous figures, the container 1 includes a guiding mechanism 6, in order to precisely guide a medical instrument 7. Moreover, reference elements 5 are arranged on the container 1. Longitudinal mesh elements 3 and transversal mesh elements 2 are provided, wherein apertures 4 are arranged between the mesh elements.

Fig. 6 shows schematically a container 1 which includes 3 container parts 1.1, 1.2, 1.3 in a perspective view. In Fig. 6, the parts 1.1, 1.2, 1.3 are separated. Dotted lines indicate how the parts 1.1, 1.2, 1.3 get assembled together, wherein any type of fittings may be arranged in order to firmly assemble the parts 1.1, 1.2, 1.3 together.

In summary, it is to be noted that a method and a system for improving the efficiency during a medical intervention has been described, wherein a container for an organ is manufactured in order to improve the efficiency during the medical intervention, in particular to improve the precision of the medical intervention and to reduce the time needed to perform the medical intervention.

### List of reference symbols

| | |
|---|---|
| 1 | Container |
| 1.1,1.2,1.3,1.4 | Parts of the container |
| 2 | Transversal elements |
| 3 | Longitudinal elements |
| 4 | Aperture |
| 5 | Reference elements |
| 6 | Guiding mechanism |
| 7 | Surgical instrument |

## Claims

1. Method for manufacturing a container (1) for an organ, comprising:
d) collecting an image data set of the organ using a medical image acquisition apparatus, in particular a CT apparatus (CT: computer tomography), a MRI apparatus (MRI: magnetic resonance imaging), and/or an ultrasonography apparatus;
e) determining a surface shape of the organ or parts thereof by analyzing the collected image data set; and
f) manufacturing the container (1) in accordance to the determined surface shape of the organ, such that the organ as a whole or only partially may be stabilized and shaped within the container (1) during a medical intervention.

2. Method according to claim 1, **characterized in that** several image data sets of organs of several persons are collected, wherein standardized surface shapes of organs are determined and containers of standardized and predefined sizes and/or shapes are manufactured accordingly, such that a container of a certain size and/or shape may be selected in order to stabilize the organ of a certain person during a surgery.

3. Method according to claim 1 or 2, **characterized in that** manufacturing the container includes a conventional or rapid prototyping process, including but not limited to laser sintering, vacuum casting, stereolithography, 3D printing and/or fused deposition modelling.

4. Method according to one of claims 1 to 3, **characterized in that** the container is manufactured in a biocompatible material, in particular a metallic material or a polymer.

5. Method according to one of claims 1 to 4, **characterized in that** the surface shape of the organ is reduced to a mesh, wherein a corresponding container (1) is manufactured in a mesh structure (2, 3).

6. Method according to one of claims 1 to 5, **characterized in that** the container (1) is manufactured such that only parts of the organ are covered and/or that the container (1) is manufactured such that extra openings for accessing the organ during a medical intervention are provided.

7. Method according to one of claims 1 to 6, **characterized in that** the container (1) is manufactured such that it includes several parts (1.1, 1.2, 1.3, 1.4), which are connectable with one or more adapters or fittings (9).

8. Method according to one of claims 1 to 7, **characterized in that** the container (1) is manufactured such that it includes reference elements (5) which are detectable with a surgical instrument guidance system, in particular in order to monitor the position and/or orientation of a surgical instrument (7) relative to the container (1) and therefore relative to the organ arranged in the container (1) during a medical intervention.

9. Method according to one of claims 1 to 8, **characterized in that** the container (1) is manufactured such that it includes one or more guiding mechanisms (6) for surgical instruments (7) and/or for diagnostic equipment.

10. System for manufacturing a container (1) for an organ, comprising:
d) an image data acquisition apparatus designed to collect an image data set of the organ, in particular a CT apparatus (CT: computer tomography), a MRI apparatus (MRI: magnetic resonance imaging), and/or an ultrasonography apparatus;
e) a surface shape determination module for determining a surface shape of the organ or parts thereof by analyzing the collected image data set; and
f) a manufacturing apparatus for manufacturing the container in accordance to the determined surface shape of the organ, such that the organ as a whole or only partially may be stabilized and shaped within the container during a surgery.

11. System according to claim 10, **characterized in that** the image data acquisition apparatus, the surface shape determination module and the manufacturing apparatus are designed such that several image data sets of organs of several persons are collected, wherein standardized surface shapes of organs are determined and containers of standardized and predefined sizes and/or shapes are manufactured accordingly, such that a container (1) of a certain size and/or shape may be selected in order to stabilize the organ of a certain person during a surgery.

12. System according to claim 10 or 11, **characterized in that** the manufacturing apparatus is designed to manufacture the container (1) in a conventional or rapid prototyping process, including but not limited to laser sintering, vacuum casting, stereolithography, 3D printing and/or fused deposition modelling.

13. System according to one of claims 10 to 12, **characterized in that** the manufacturing apparatus is designed to manufacture the container (1) such that it includes several parts (1.1, 1.2, 1.3, 1.4), which are connectable with one or more adapters or fittings (9).

14. System according to one of claims 10 to 13, **characterized in that** the manufacturing apparatus is designed to manufacture the container (1) such that it includes reference elements (5) which are detectable with a surgical instrument guidance system, in particular in order to monitor the position and/or orientation of a surgical instrument (7) relative to the container (1) and therefore relative to the organ arranged in the container (1) during a medical intervention.

15. System according to one of claims 10 to 14, **characterized in that** the manufacturing apparatus is designed to manufacture the container (1) such that it includes one or more guiding mechanisms (6) for surgical instruments (7) and/or for diagnostic equipment.
